# EUROPEAN PATENT APPLICATION

(11) **EP 4 193 918 A1**
(43) Date of publication of application: **14.06.2023**
(21) Application number: 21212753.4
(22) Date of filing: 07.12.2021
(51) Int. Cl.: A61B 5/0536, A61B 5/00, A61B 5/0205, A61B 5/11

(54) **APPARATUS, METHODS AND COMPUTER PROGRAMS FOR IDENTIFYING CHARACTERISTICS OF BIOLOGICAL SAMPLES**

(71) Applicant: Nokia Technologies Oy, 02610 Espoo (FI)
(72) Inventor: ZHENG, Mingde, Basking Ridge, NJ, 08807 (US); JAHANANDISH, Hassan, Dallas, TX, 75252 (US)
(74) Representative: Nokia EPO representatives

(57) **Abstract**

Examples of the disclosure relate to apparatus, methods and computer programs for identifying characteristics of biological samples. The apparatus can comprise means for obtaining a plurality of multi-dimensional and multi-layered datasets from a biological sample and dividing the plurality of multi-dimensional and multi-layered datasets into a plurality of sections wherein each section comprises at least one feature. The means are also for filtering, for at least a subset of the sections, the plurality of multi-dimensional and multi-layered datasets to select features of interest, comparing the selected features of interest to a plurality of reference features and making one or more associations between two or more selected features of interest to identify one or more characteristics of the biological sample.

## Description

### TECHNOLOGICAL FIELD

Examples of the disclosure relate to apparatus, methods and computer programs for identifying characteristics of biological samples. Some relate to apparatus, methods and computer programs for identifying characteristics of biological samples in-vivo.

### BACKGROUND

Analysis of biological samples can provide useful information about the biological sample. The information that is obtained can be used for controlling an electronic device, performing diagnosis, health monitoring or any other suitable purpose.

### BRIEF SUMMARY

According to various, but not necessarily all, examples of the disclosure, there is provided an apparatus comprising means for:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

The plurality of multi-dimensional and multi-layered datasets may be obtained from an output signal provided by stimulating the biological sample with one or more of: an electrical signal, an acoustic signal, an electromagnetic signal so as to cause changes in electrical properties of the biological sample.

The plurality of multi-dimensional and multi-layered datasets may comprise electrical impedance tomography signals.

The reference features may be determined from calibration outputs from a reference biological sample.

The reference features that are determined from calibration outputs may be identified as corresponding to one or more characteristics of the biological sample.

At least some of the plurality of reference features may be associated with each other based on the one or more characteristics of the biological sample that the reference features correspond to.

The means may be for accessing a memory storing the reference features and information indicative of the one or more associations between the different reference features.

Different associations between different selected features of interest may enable one or more characteristics of the biological sample to be identified.

The reference features may be given identifiers that are linked to characteristics of the biological sample and in response to identifying a match between a respective selected feature and a respective reference feature, using the identifiers to identify the one or more characteristics of the biological sample.

The apparatus may comprise means for dynamically tuning one or more filter settings for filtering the plurality of multi-dimensional and multi-layered datasets to select features of interest.

The apparatus may comprise means for autonomously learning and storing reference features by operating the filters iteratively on labelled reference features.

The means may be for processing at least some of the plurality of sections of the datasets in parallel, wherein the processing comprises filtering a plurality of sections of the datasets to select features of interest.

The parallel processing of the plurality of sections may enable a plurality of different features of interest to be selected simultaneously.

The plurality of multi-dimensional and multi-layered datasets may vary over time and the means are for filtering the plurality of multi-dimensional and multi-layered datasets over a period of time.

The characteristic of the biological sample that is identified may comprise one or more of: the position of one or more components within the biological sample, movement of one or more components within the biological sample, the size of one or more components within the biological sample, the conductivity of one or more components within the biological sample, an electrical parameter of one or more components within the biological sample or the shape of one or more components within the biological sample.

According to various, but not necessarily all, examples of the disclosure, there is provided a method comprising:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

According to various, but not necessarily all, examples of the disclosure, there is provided a computer program comprising computer program instructions that, when executed by processing circuitry, cause:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

### BRIEF DESCRIPTION

Some examples will now be described with reference to the accompanying drawings in which:
FIG. 1 shows an example apparatus;
FIG. 2 shows an example method;
FIG. 3 shows an example method;
FIG. 4 shows examples datasets;
FIGS. 5A to 5D shows a relation between characteristics of a biological sample and datasets;
FIG. 6 shows an example method;
FIG. 7 shows an example method;
FIGS. 8A and 8B show a relation between selected features and biological characteristics;
FIG. 9 shows an example method;
FIG. 10 shows an example method; and
FIG. 11 shows an example apparatus.

### DETAILED DESCRIPTION

Examples of the disclosure relate to systems and methods for identifying characteristics of biological samples. The identified characteristics can be used for human-machine interactive purposes, health monitoring purposes, to provide control signals to electronic devices or computer programs, for diagnostic purposes or for any other suitable use.

In examples of the disclosure a plurality of multi-dimensional and multi-layered datasets can be obtained from a biological sample. The datasets can then be divided into a plurality of sections where each section comprises at least one feature indicative of biological sample characteristics. These sections can be processed in parallel to enable one or more characteristics of the biological sample to be identified from the sections of the datasets.

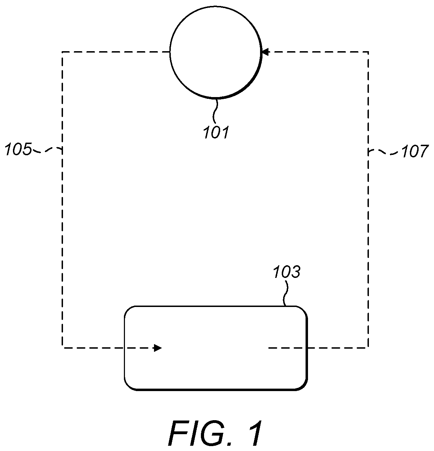

Fig. 1 schematically shows an example apparatus 101 for analysing a biological sample 103. The apparatus 101 could be any suitable interrogative device or analysis system, such as an electrical impedance tomography apparatus or other types that can be configured to analyze biological samples 103.

The biological sample 103 can comprise an in-vivo sample. The biological sample 103 can comprise any biological cellular matter. In some examples, the biological sample 103 could comprise a part of a user's body or any other suitable biological sample 103. For instance, in some examples the biological sample 103 could comprise part of a person's arm and the apparatus 101 could be configured to use methods of the disclosure to detect movement or other changes of the arm.

The apparatus 101 can be configured to enable one or more characteristics of the biological sample 103 to be identified. The characteristic could comprise an intrinsic or extrinsic property of the biological sample 103. For example, the characteristics could comprise an indication about the types of structures or component within the biological sample 103. In such cases the characteristics could comprise information about a type of cell or tissue within the biological sample 103. The characteristics could comprise information relating to the position of such cells and tissues and/or the size, shape and/or conductivities of such cells and tissues and/or electrical parameters of such cells or any other suitable information.

In some examples the characteristics could comprise changes within the biological sample 103. For example, the characteristics could comprise movement of the biological sample 103 and/or parts of the biological sample 103. In some examples the changes could comprise a change in size and/or shape and/or position of cells or tissues or other components within the biological sample 103. Other types of characteristics could be used in other examples of the disclosure.

In some examples of the disclosure an electrical input signal 105 can be provided to the biological sample 103. The electrical input signal 105 can comprise any electrical signal that can stimulate the biological sample 103 so as to elicit one or more electrical properties of the biological sample 103. The electrical input signal 105 can be configured so that it does not cause irreversible damage to the cells and other components within the biological sample 103 or does not kill the cells or other components within the biological sample 103.

The electrical properties of the biological sample 103 that are elicited can be any properties that can be measured or detected by using the electrical input signals 105. The electrical properties that are elicited could comprise any one or more of resistance, capacitance, impedance, dielectric, phase or any other suitable property. The electrical properties of the biological sample 103 that are elicited could comprise properties of cells/tissues, or parts of cells and tissues, within the biological sample 103.

The apparatus 101 can also be configured to receive electrical output signals 107 from the biological sample 103. The electrical output signals 107 correspond to the electrical input signals 105 in that they can be provided by the electrical input signals 105 passing through the biological sample 103. As the electrical output signals 107 comprise signals that have passed through the biological sample 103, the properties of the electrical output signals 107 are determined by the electrical properties of the biological sample 103. This enables the electrical output signals 107 to provide information relating to the electrical properties of the cells within the biological sample 103 and so can provide information relating to one or more characteristics of the biological sample 103.

The apparatus 101 comprises means for enabling analysis of the electrical output signals 107. The analysis of the output signals 107 can enable particular types of characteristics to be identified and/or monitored. In some examples the analysis of the output signals can enable full or combinatorial characteristics of the biological sample 103 to be identified. Example methods that can be used for the analysis of the electrical output signals 107 are described below.

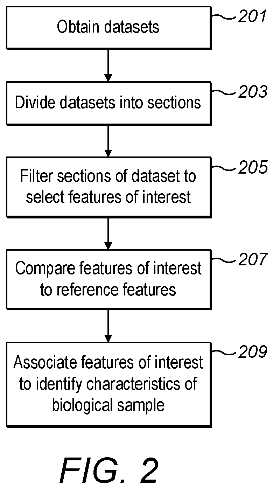

Fig. 2 shows a method that can be implemented using an apparatus 101 as shown in Fig. 1. The method enables information to be obtained from a biological sample 103 such that the information can be used to identify characteristics of the biological sample 103.

The method comprises; at block 201, obtaining a plurality of multi-dimensional and multi-layered datasets from a biological sample 103. The datasets can comprise any sets of data that can be processed as a unit by the apparatus 101. The datasets can be configured to be divided into smaller elements or sections as needed. The datasets can represent waveforms. In some examples the datasets can comprise data obtained from waveforms.

The datasets can be obtained from an output signal where the output signal is provided by stimulating the biological sample 103 with an appropriate input signal or stimulation signal. The biological sample 103 can be stimulated so as to cause changes in electrical properties of biological sample 103. In some examples the biological sample 103 can be stimulated so as to cause changes in electrical properties of one or more elements within the biological sample 103. The biological sample 103 can be stimulated using any one or more of; an electrical signal, an acoustic signal, an electromagnetic signal or any other suitable type of signal.

In some examples the datasets can be obtained from different parts of a biological sample 103. For example, electrodes could be positioned to different parts of a biological sample to enable the output signals to be obtained from different parts. This can enable different information to be provided in different output signals from different parts of the biological sample 103.

In some examples the plurality of multi-dimensional and multi-layered datasets comprise electrical impedance tomography signals. Other types of signals and/or datasets could be used in other examples.

The datasets can be multi-dimensional in that the datasets comprise information relating to time and space. The datasets can be multi-layered in that they comprise information relating to a plurality of different features. The different features can enable one or more characteristics of the biological sample 103 to be identified. In some examples the different features can be analysed by comparing them to reference features. This can enable particular types of characteristics of the biological sample 103 to be identified and/or monitored and or it can enable full or combinatorial characteristics of the biological sample 103 to be identified.

At block 203 the method comprises dividing the plurality of multi-dimensional and multi-layered datasets into a plurality of sections. The datasets can be divided so that each section comprises at least one feature.

At block 205, at least a subset of the sections of the datasets are filtered to select features of interest. The features of interest can be features relating to characteristics of the biological sample 103 that are to be monitored or identified. The filters that are used to filter the sections of the datasets can be selected and/or adapted based on the features of interest that are to be selected.

Different filters can be used to select different features of interest. For example, a first filter can be used to select a first feature and discard a second and any other feature and a second, different filter could be used to select a second feature and discard the first and any other feature.

In some examples the settings of the filters that are used for filtering the sections of the datasets can be dynamically tuned. The dynamic tuning can be used to enable different features of interest to be selected. For example, the filters can be tuned to focus on features of interest that are user-defined to be significant and/or can be tuned to discard information relating to features that are not of interest. This can enable the filters to provide attention filters where the attention can be focused on features of interest.

The filters that are used can improve the accuracy with which the characteristics of the biological sample 103 can be identified. By removing the features that are not of interest, this not only reduces the dataset size that needs to be analysed, but also ensures that the processes and algorithms are applied to the key features of the dataset.

The filters that are used can be configured to provide different levels of matching degrees to the desired features of interest. The different levels of matching can enable the most relevant characteristics of the biological sample 103 to be identified with different levels of certainty. This can enable characteristics of the biological sample 103 to be identified in the absence of absolute certainty. In such examples a matching percentage for each filtering operation can be calculated. The selection of a feature can be based on this matching percentage. For example, if a matching percentage is 100 or close to 100 this can be considered to be an identical match. If there is no identical match then the feature with the highest matching percentage could be selected as a best match.

At block 207 the method comprises comparing the selected features of interest to a plurality of reference features. In some examples the reference features are determined from calibration outputs from a biological sample 103. The reference features could be obtained through in-vivo and in-vitro measurements of a biological sample 103 or from simulations or by any other suitable means.

The reference features that are determined from calibration outputs from a biological sample 103 can be identified as corresponding to one or more characteristics of the biological sample 103. Any suitable means can be used to identify the characteristics of the biological sample 103 that correspond to the reference features in the calibration outputs. In some examples machine learning or any other adaptive algorithms could be used to identify the characteristics of the biological sample 103 that correspond to the reference features in the calibration outputs. For example, the characteristics of the biological sample 103 can be identified by autonomously learning and storing reference features by operating the filters iteratively on labelled reference features.

In some examples calibration outputs can be obtained from a first type of biological sample 103 and used for other biological samples 103 of the same type. For instance, calibration outputs could be obtained from a user's hand and then used for the hands of other users.

At least some of the plurality of reference features can be associated with each other. In some examples, two or more reference features can be associated with each other based on the one or more characteristics of the biological sample 103 that the reference features correspond to. For example, reference features relating to a user's finger could be associated with reference features relating to other fingers or other parts of the hand. This is because movement of one finger could cause changes in characteristics of other parts of the biological sample 103 such as the fingers and/or the hand. Also, if a user is making a recognisable hand gesture, this could comprise pointing their fingers and/or parts of their hand in a particular way.

At least some of the plurality of reference features can be associated with one or more other plurality of reference features based on the one or more characteristics of the biological sample 103 that the reference features correspond to. For example, reference features relating to fingers could be associated together and/or reference features relating to parts of the arm could be associated together. The associations between the reference features can comprise connections or links as retrievable information or within a digital memory that can be accessed by the apparatus 101.

At block 209 the method comprises making one or more associations between two or more selected features of interest. This can enable interconnections or relationships between the features of interest to be analysed.

In some examples the associations between two or more selected features of interest can be made by accessing and analysing one or more memory storing the reference features and the connections or links to other reference features. This can enable relationships or interconnections between features of interest to be identified.

The associations between the two or more selected features of interest can be used to identify one or more characteristics of the biological sample 103. Different associations between different selected features of interest enable different characteristics of the biological sample 103 to be identified. Any suitable means can be used to identify the one or more characteristics of the biological sample 103. In some examples a decision tree could be used to identify the one or more characteristics of the biological sample 103.

In some examples the reference features, or associations of reference features, can be given identifiers. The identifiers can enable the reference features, or associations of reference features, to be linked to characteristics of the biological sample 103. When a match is identified between a respective selected feature and a respective reference feature, the identifiers can be used to identify the one or more characteristics of the biological sample 103.

The characteristic of the biological sample 103 that is identified can comprise any suitable characteristic. In some examples the characteristic can comprise the position of one or more components within the biological sample 103. For instance, the position of muscles or bones within a user's hand could indicate a position of the user's fingers and whether or not they are making a particular gesture. In some examples the characteristic could comprise movement of one or more components within the biological sample 103. For instance, detecting a change in the position of one or more muscles within a biological sample could indicate that a user is making a gesture or is about to make a gesture. In some examples the characteristic could comprise the size of one or more components within the biological sample 103. For instance, the size of muscles or blood vessels or any other suitable components within the biological sample 103 could be monitored. The size of one or more components could also be useful for diagnostic purposes. In some examples the characteristic could comprise the conductivity or other electrical parameter of one or more components within the biological sample 103. The conductivity or other electrical parameter of particular components could indicate that the user is performing a particular gesture or could indicate information relating to a user's health or physical condition. In some examples the characteristic could comprise the shape of one or more components within the biological sample 103. For instance, the shape of particular components could indicate that the user is performing a particular gesture or could indicate information relating to a user's health or physical condition. Other characteristics could be identified and/or monitored in other examples of the disclosure.

In some examples the processing of at least some of the plurality of sections can be performed in parallel. The processing can comprise the filtering of the plurality of sections to select features of interest, comparing the selected features to the reference features and associating features of interest to identify characteristics of the biological sample 103 or any other suitable processing. The processing can comprise assigning indices to sections of the datasets. The different indices can enable different sections of the datasets to be tracked so that they can be processed in parallel. The different indices can relate to different periods of time and/or spatial areas.

The parallel processing of the plurality of sections enables a plurality of different features of interest to be selected simultaneously. The features of interest that have been selected simultaneously can then be compared to reference features and associated with other features simultaneously. The processes and algorithms can be applied to smaller sets of the data at the same time as opposed to applying the processes and algorithms to a single larger dataset. This can reduce the time it takes to identify the one or more characteristics of the biological sample 103. This can reduce the latency in the time it takes for the characteristics of the biological sample 103 to be identified.

In some examples the plurality of datasets can vary over time. In such examples the apparatus can be configured to filter the datasets over a period of time. The use of the parallel processing can reduce the delay providing the indication of the characteristic of the biological sample 103.

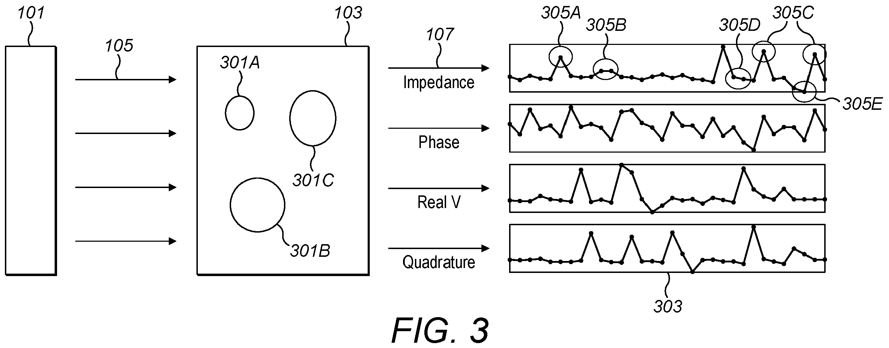

Fig. 3 schematically shows an example method that can be used in some examples of the disclosure.

In the example of Fig. 3 an apparatus 101 is configured to provide a plurality of electrical input signals 105 to a biological sample 103.

The electrical input signals 105 are provided to the biological sample 103. In this example the biological sample 103 comprises three different elements 301A-301C. the different elements 301A-301C can be parts of the biological sample 103 that comprise physical properties that change over time. The different elements 301A-301C could be different muscles or other tissues within the biological sample 103 or any other suitable parts of the biological sample 103. The different elements 301A-301C are shown schematically in Fig. 3.

The electrical input signals 105 can change the electrical properties of the elements 301A-301C and/or the parts of the biological sample 103 around the elements 301A-301C. When an electrical input signal 105 is provided to the biological sample 103 this will pass through the elements 301A-301C to provide the electrical output signals 107. The reactions or responses from the electrical properties of the 301A-301C therefore determine the values of the electrical output signals 107.

Fig. 3 also shows a plurality of waveforms 303 indicative of the electrical output signals 107. The waveforms 303 provide a plurality of multi-dimensional and multi-layered datasets from a biological sample. In some examples each of the different waveforms 303 can provide a different dataset.

The waveforms 303 shown in Fig. 3 show values of different parameters of the electrical output signals 107. In the example of Fig. 3 the waveforms 303 show values of impedance, phase, voltage and quadrature. The vertical axis of the waveforms 303 indicate these values. Other parameters could be used in other examples of the disclosure.

The horizontal axis of these waveforms 303 correspond to the different observations or measurements made by different pairs of electrodes that have been coupled to the biological sample 103. The different pairs of electrodes can correspond to different parts of the biological sample 103 and/or to different probing directions of the biological sample 103. In this example, each of the waveforms 303 comprise thirty-two different measurements along the horizontal axis. These measurements are obtained using different pairs of electrodes to provide electrical input signals 105 and electrical output signals 107. Other numbers of measurements and/or arrangements for the measurements could be used in other examples of the disclosure.

The waveforms 303 comprise a plurality of features 305A-305E. The features 305A-305E comprise measurement points and/or groups of measurement points that can be easily identified within the waveforms 303. For example, the features can comprise the global maxima of one or more parameters within the waveforms 303. In some examples features could comprise local maxima or local minima, or any other suitable type of measurement points.

The different features 305 within the waveforms 303 can be associated with different elements or other characteristics of the biological sample 103. For example, each of the elements 301A-301C in the biological sample 103 shown in Fig. 3 could be associated with different features in the resulting waveforms 303. The features 305A-305E can provide an indication about the shape, position or other parameters of the elements 301A-301C. The combination of these different features 305A-305E can therefore be used to identify patterns of movement or other types of changes or alterations of the elements 301A-301C. This can therefore be used to identify characteristics of a biological sample 103 such as a gesture being performed.

In order to identify one or more characteristics of the biological sample 103 the features 305A-305E within the waveforms 303 can be compared to one or more reference features. In examples of the disclosure the waveforms 303 or datasets representing the waveforms 303 can be divided into a plurality of sections where each of the sections comprise at least one feature.

The divided sections of the waveforms 303 or datasets representing the waveforms can be filtered to enable features of interest to be selected. The filtering can select features that are to be compared with the reference features. The filtering can enable unwanted features to be discarded. The discarded features are not compared with the reference features. This focusses the attention on the features of interest and reduces the processing that is required.

The features that comprise the features of interest can be determined in response to a user input. For instance, a user could indicate that they want to monitor certain one or more characteristics of the biological sample 103. In such cases the features corresponding to those one or more characteristics would become the features of interest.

In some examples the features that comprise the features of interest could be determined in response to a different input. For example, if the apparatus is being used as an interface to control another device this could indicate that the apparatus 101 needs to be configured to detect particular gestures. Therefore, the applications that are being controlled by the apparatus, and the gestures that can be used to enable the control, can determine the features of interest.

The features of interest that are selected by the filtering can be compared to reference features. The reference features used for the comparison can be obtained from experimental results, from simulations, in-vitro observations or by any other suitable means. The comparison with the reference features can provide an indication as to whether or not the selected feature corresponds to the reference feature. For instance, it provides an indication as to whether or not the selected features correspond to the same element or characteristic of the biological sample 103.

Associations can be made between features of interest. The associations can comprise a link that enable further information about the selected feature to be obtained and/or enables more accurate information about the selected feature to be obtained. For instance, in some examples the association can enable two or more features to be linked together to provide an indication of a gesture or other status or characteristic of a biological sample 103.

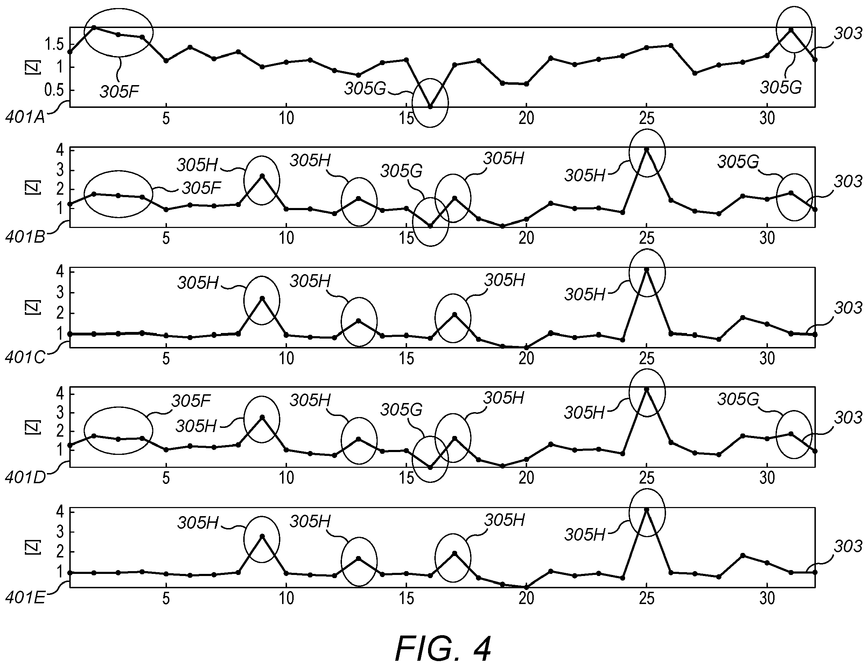

Fig. 4 shows example datasets 401 that can be used in some examples of the disclosure. The datasets 401 comprise data from a plurality of waveforms 303. In this example the waveforms 303 and datasets 401 are obtained from output signals obtained by providing electrical input signals to stimulate a biological sample 103. Other types of signals can be used in other examples of the disclosure.

Each of the datasets 401 in the example of Fig. 4 are obtained from different waveforms 303. The different waveforms 303 in the example can be obtained from different portions of the biological sample 103. In some examples the different waveforms 303 can be obtained at different times. In some examples the datasets 401 can comprise data that is obtained both from different portions of the biological sample 103 and at different times.

The datasets 401 are multidimensional in that they comprise features that are dependent on both time and space. This can enable different features to be identified from different positions and/or at different times. For examples different electrodes or measure points can be positioned at different locations on the biological sample 103 to enable information from different positions to be obtained. The information at different times can be obtained by continuously monitoring the biological sample over a period of time and/or by obtaining measurements at different time instances.

The datasets 401 are multi-layered in that they comprise information relating to a plurality of different features 305. The plurality of different features 305 can relate to different elements and/or characteristics of the biological sample. This means that each dataset comprises information that can be used to enable a plurality of different elements and/or characteristics of the biological sample to be identified.

In the example of Fig. 4, five datasets 401A-E are shown. Each of the datasets comprise different combinations of features. In the example of Fig. 4 the first dataset 401A, the second dataset 401B and the fourth dataset 401D comprise a first feature 305F. The second dataset 401B and the fourth dataset 401D comprise a second feature 305H. The first dataset 401A, second dataset 401B, third dataset 401C and fourth dataset 401D comprise a plurality of third features 305G.

The size and shape of the features 305F-H can vary at different points in time depending on changes within the biological sample 103 and/or depending upon the position that they are measured from. Comparing these features to reference features can enable them to be identified as corresponding to particular elements of the biological sample 103. Associations between the features 305 can be used to enable characteristics of the biological sample 103 to be identified.

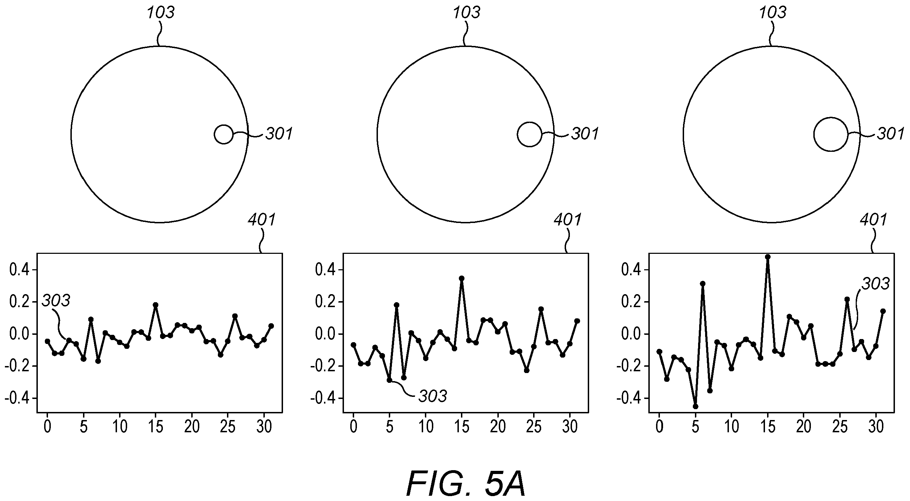

Figs. 5A to 5D show relations between characteristics of a biological sample 103 and datasets 401. In this example the datasets represent waveforms 303 or data points obtained from waveforms 303. The waveforms 303 can be obtained from output signals 107 as shown in Figs. 1 and 3 or from any other suitable types of signal.

Figs. 5A to 5D also schematically show part of a biological sample 103. This biological sample 103 comprises an element 301. The biological sample 103 and element 301 are shown schematically in Figs. 5A to 5D. The biological sample 103 and element 301 could be any suitable biological sample 103 and element 301.

The example of Fig. 5A shows how different patterns of the element 301 affect the different features within the waveform 303. Fig. 5A shows three different examples with an element 301 of three different sizes. In this example waveform 303 comprises the same features for each of the different sizes. However, the amplitude of the features has increased as the size of the element 301 increases.

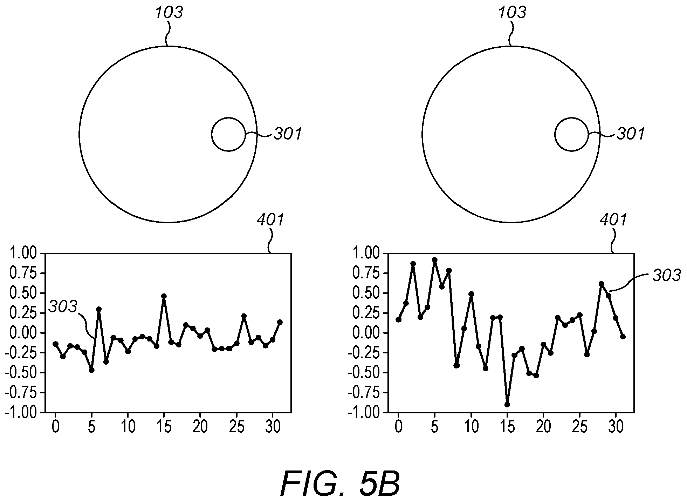

Fig. 5B shows how different conductivity of the element 301 affects the different feature within the waveform 303. The conductivity of the element 301 could be due to the type of element 301 so that different types of element within a biological sample 103 show different types of conductivity. In some examples the conductivity of the element 301 could change as an input signal 105 or other stimulating signal is applied to the biological sample 103. In the example of Fig. 5B this results in a change in patterns of the features of the waveform 103.

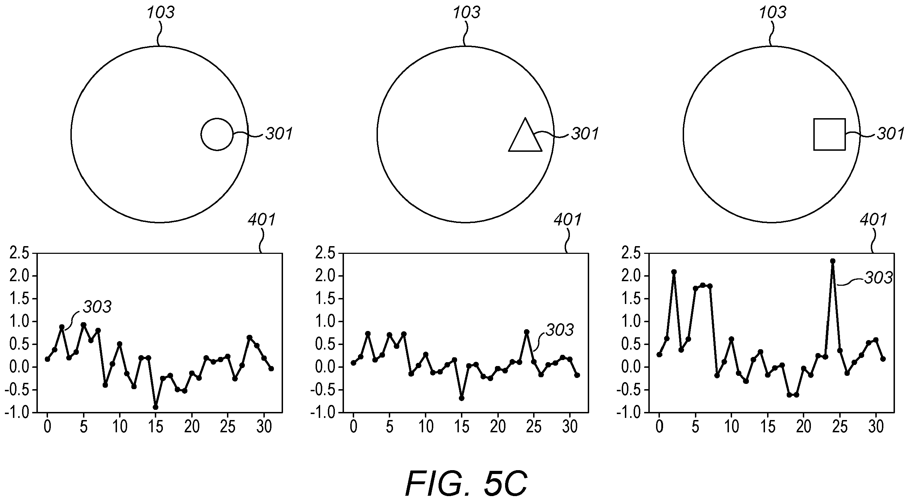

Fig. 5C shows how different patterns of the element 301 affects the different features within the waveform 303. In the example of Fig. 5C the different shapes of the element 301 results in a change in patterns of the features of the waveform 103.

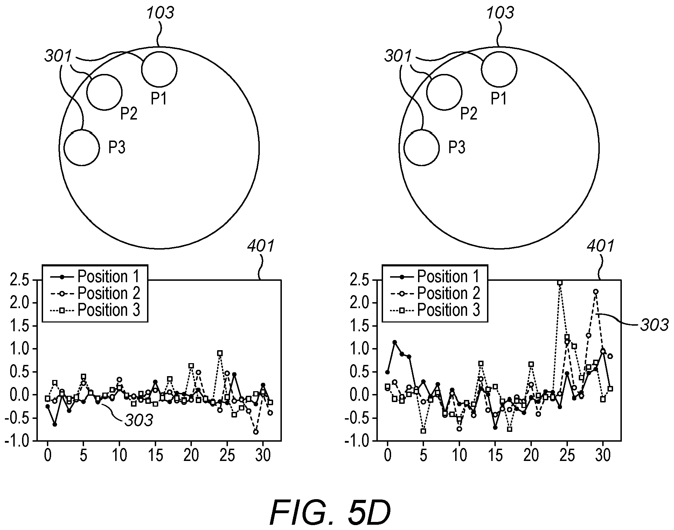

Fig. 5D shows how different positions of the element 301 affect the different features within the waveform 301. In the example of Fig. 5D the element can move from a first position to a second position and from a second position to a third position. This creates three different waveforms 303. The different waveforms 303 can be obtained from the biological sample 103 at different time instances. In the example of Fig. 5D the different elements have different conductivities. This generates different groups of waveforms 303 depending upon the position and the conductivity of the element 301.

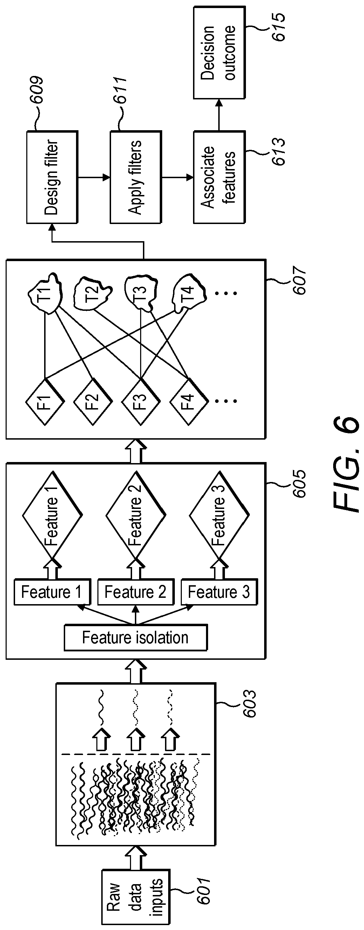

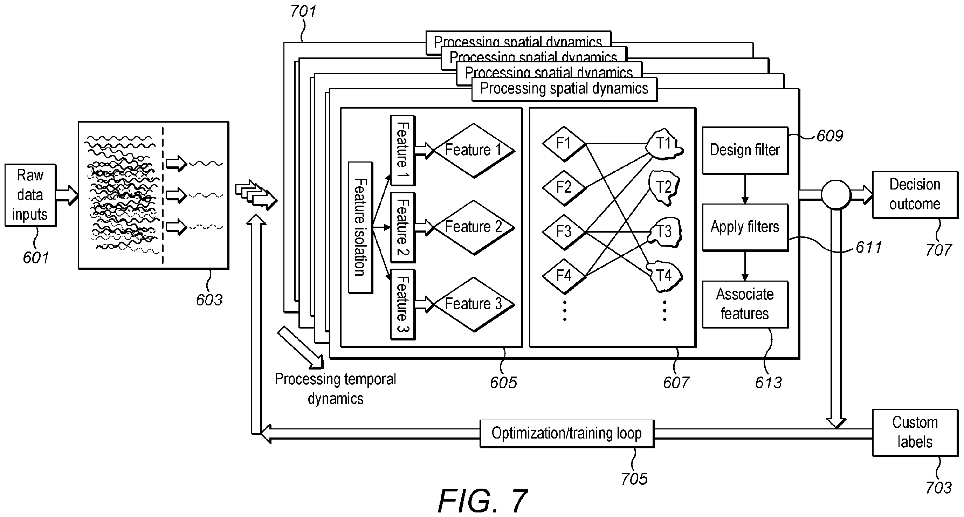

Fig. 6 shows an example method that can be used to implement some examples of the disclosure. This method can be used to calibrate an apparatus 101 by using reference features obtained from a reference biological sample. The reference biological sample 103 could be the same type and/or species of biological sample 103 that the apparatus 101 is to be used upon. The output of the method can provide a set of reference features that can be used to implement examples of the disclosure such as being input to the training or customization iterations loop 705 shown in Fig. 7.

The method comprises, at block 601, obtaining raw data inputs. The raw data inputs can comprise unprocessed data from the biological sample 103. The raw data inputs can comprise a plurality of output signals 107 obtained from a biological sample 103 or could comprise output from a simulation or any other suitable type of raw data. The raw data inputs can comprise a plurality of waveforms or datasets or any other suitable input.

At block 603 background data and interference data is eliminated from the raw inputs. This allows datasets relating to the biological sample 103 to be extracted from the raw inputs and used for processing. The datasets can comprise multi-dimensional and multi-layered datasets.

Any suitable process can be used to eliminate the background data and interference data from the raw inputs. For example, digital signal processing methods and/or conditioning algorithms can be used to remove the background data and interference data.

At block 605 a plurality of features can be isolated within the datasets. The isolation of the features can comprise finding patterns within the datasets that correspond to features. The patterns could comprise features with global maxima or minima within the waveforms, local maxima or local minima, or any other suitable type of measurement points within the waveforms.

In some examples the isolated features can be converted into a format suitable for processing. For instance, the features can be converted into a vector format, an array format or any other suitable type of format. The type of format that is used for the features can depend on the type of processing that is to be performed on the features, the algorithms and/or processes used to process the features of the datasets.

At block 607 a connection between the features and one or more elements of the biological sample 103 can be established. The connection can be established by retrieving information from a digital storage bank. The information in the digital storage bank can comprise information that links the features to the corresponding elements or characteristics of the biological sample 103. For example, the features that have been isolated can be linked to a particular type of tissue within the biological sample 103, a particular section of tissues within the biological sample 103 or any other suitable part of the biological sample.

Any suitable means can be used to establish a connection between the features that have been isolated and one or more elements of the biological sample 103. In some examples the connection can be established by comparing the features to reference features. The reference features can be obtained by calibration measurements or by any other suitable process.

In some examples recursive learning can be used to establish the connections between the features that have been isolated and one or more elements of the biological sample 103.

At block 609 one or more filters can be designed. The filters can be designed to enable one or more features of interest to be selected from the available features in the datasets. A filter can be designed to select the features that have linked to particular elements of a biological sample. For instance, if it has been identified that certain features are associated with a specific tissue characteristic of the biological sample 103 then a filter can be designed to select those features and discard the other features. This can enable the selection of features that are associated with that specific tissue characteristic. The filter designed to select these features can therefore be used to identify characteristics of the biological sample 103 that involve that tissue.

The process of linking the features to components and designing the filters can be performed whenever appropriate. In some examples this part of the process might be performed once to enable the appropriate filters to be designed.

At block 611 one or more filters are applied. The filter that is applied can be a filter that has been designed at block 609. In some examples the filter that is applied can be a filter that has been designed previously and can be retrieved from an appropriate digital storage bank.

The filters that are applied can be chosen to enable particular features of interest to be selected. For instance, if the process is to be used to look for particular gestures or features of a biological sample 103 the filters can be chosen to select the features that have been linked to elements of the biological sample 103 that are involved with those gestures or features.

The application of the filters at block 611 enables a plurality of features of interest to be selected. This reduces the features of interest that are to be processed.

At block 613 associations can be made between the selected features of interest. The associations can be used to identify one or more characteristics of the biological sample 103. Different associations between different selected features of interest enable different characteristics of the biological sample to be identified. For example, an association between a first feature and a second feature can be indicative of a first characteristic of the biological sample while an association between the first feature and a third feature can be indicative of a second characteristic of the biological sample.

The associations can help to increase the accuracy and/or efficiency with which the characteristics of the biological sample 103 can be identified. A library of associations can be generated and stored in a digital storage bank and used to identify characteristics of the biological sample 103.

At block 615 the method comprises providing a decision outcome. The decision outcome enables classification of the associations of the features of interest. The classification can comprise classifying a particular association of features of interest with a particular characteristic of a biological sample 103. For instance, an association between a first feature of interest and a second feature of interest can be classified as a first characteristic of the biological sample 103 while an association between the first feature of interest and a third feature of interest can be classified as a second characteristic of the biological sample 103.

The different characteristics of the biological sample 103 could be any recognisable characteristics. For instance, they could comprise a size change of a particular tissue, a position change of a particular tissue, a conductivity change of a particular tissue, movement of a part of the biological sample or any other suitable characteristics or combinations of the characteristics.

Fig. 7 shows another example method that can be used to implement some examples of the disclosure. This method can also be used to calibrate an apparatus 101 for use with a particular type and/or species of biological sample 103. For example, the method can provide a set of reference features that can be used to implement examples of the disclosure such as being input to the training or customization iterations loop 705.

This method of Fig. 7 comprises some of the blocks of the method of Fig. 6. Corresponding reference numerals are used for corresponding features.

The method of Fig. 7 comprises, at block 601, obtaining raw data inputs and at block 603 eliminating background data and interference data from the raw data inputs. Blocks 601 and 603 can be the same as, or similar to, blocks 601 and 603 as shown in Fig. 6.

The method of Fig. 7 also comprises a plurality of spatial dynamic blocks 701. The spatial dynamic blocks comprise blocks 605 to 613 which can be the same as or similar to blocks 605 to 613 of Fig. 6. These blocks can be repeated at different times using raw data that has been obtained at different times.

Once the associations between features have been generated by a spatial dynamic block 701 a custom label can be assigned to the output. The custom labels can be used to enable the associations for a particular time period to be identified.

The custom labels can then be provided as inputs for training or customization iterations loop 705. This can enable the processes in spatial dynamic block 701 to be repeated using updated associations between features as a reference.

At block 707 the method comprises providing a decision outcome. The decision outcome enables classification of the associations of the features of interest. The classification can comprise classifying a particular association of features of interest with a particular characteristic of a biological sample 103. This classification can be based on raw data obtained at different times.

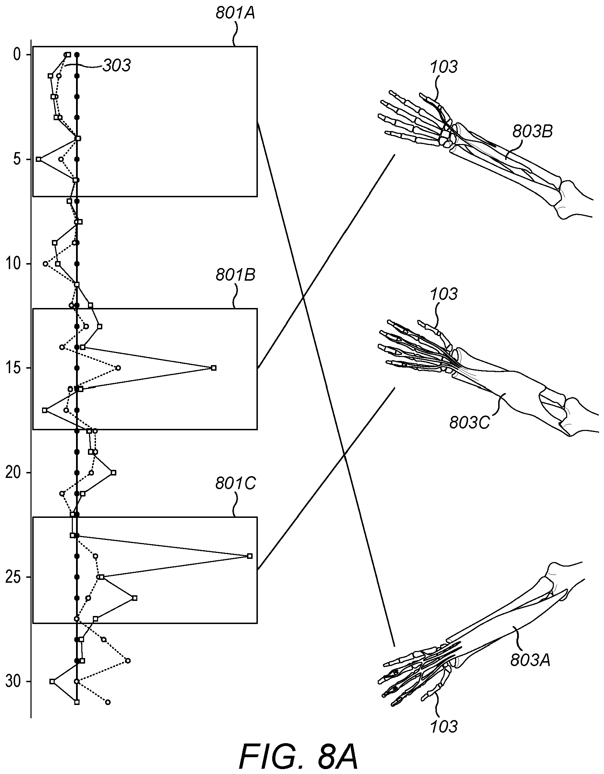

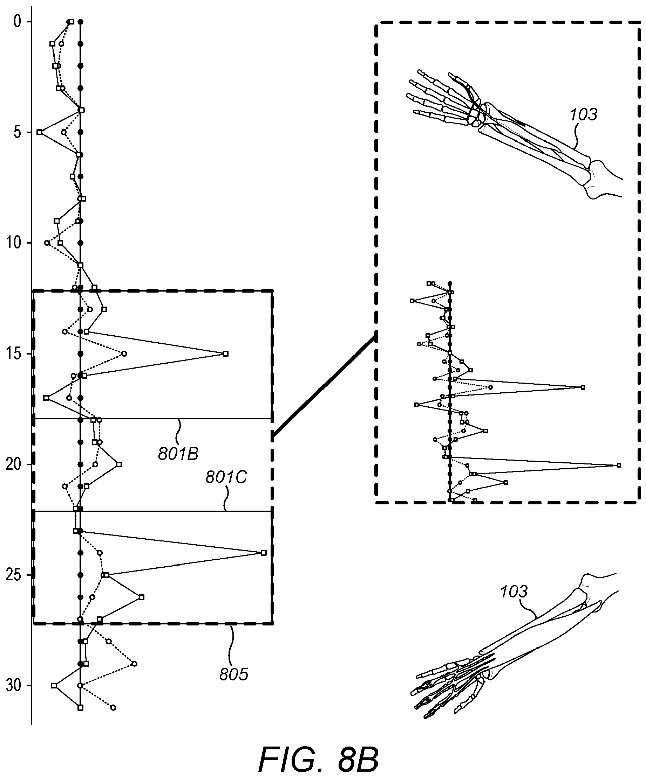

Figs. 8A and 8B show a relation between selected features and biological characteristics.

Fig. 8A shows a plurality of waveforms 303 that provide a plurality of multi-dimensional and multi-layered datasets. The plurality of waveforms 303 can be obtained by stimulating a biological sample 103. In this example the biological sample 103 comprises a user's forearm and hand. Other types of biological sample 103 can be used in other examples of the disclosure.

The waveforms 303 comprise a plurality of different features 801A, 801 B and 801C. In this example each of the features 801A, 801 B and 801C comprise a group of measurement points that can be identified within the waveforms 303. Other types of features could be used in other examples. For example, a feature could comprise a single measurement point rather than a group of measurement points.

In the example of Fig. 8A the waveforms 303 comprise three different features 801A, 801B and 801C. Other numbers of features 801A, 801B and 801C could be provided within the waveforms 303 in other examples.

The different features 801A, 801B and 801C can be linked to different components of the biological sample 103. In this example the different components within the biological sample 103 comprise different muscles 803A, 803B, 803C. In this example the first feature 801A is associated with finger extensor muscles 803B, the second feature 801B can be associated with the thumb extensor muscles 803B, and the third feature 801C can be associated with the finger flexor muscles 803C.

The links between the different features 801A, 801B, 801C and the different types of muscles can be made using calibration measurements, simulations or any other suitable process.

Fig. 8B shows an association 805 between a plurality of the features 801B and 801C. In this example the association 805 is made between the second feature 801B and the third feature 801C. In this example these are the features 801B associated with the thumb extensor muscles 803B and the features 801C associated with the finger flexor muscles 803C.

These muscles could be used when a user is making a specific gesture such as a thumbs up gesture. This gesture could be used to make an input or to control an external device or for any other suitable purpose. By associating these two features 801B, 801C together, they can be used as a pair of connected features and compared to other associations or connections between pairs of, or other group of, features. This can enable the characteristic of the user creating a thumbs up gesture to be identified with more accuracy than by considering the features 801B, 801C on their own.

In this example of Fig. 8B, the first feature 801A is missing in the waveforms 303. The absence of a feature can also be used to identify characteristics of a biological sample 103 in addition to, or instead of, information about the features that are present. For example, in Fig. 8B the first feature 801A relating to the finger extensors is absent. This can provide an indication the finger extensors are not being used. This information can be used to help to identify the characteristics of the biological sample 103.

In other examples other types of biological samples can be used and the associations between the features can be used to identify different types of characteristics. For example, other types of gestures or movements could be recognised. In some examples the characteristics could be changes or other features of the characteristics that the user is not in control of. For example, the characteristics could be used to monitor health parameters of other features of a subject.

In some examples the associations between the features can be based on features that are dependent upon each other. For instance, in the examples of Fig. 8A and 8B the movement of some of the fingers might also require or cause movement of other fingers or parts of the user's hand. Therefore, if one muscle group is used it might be expected that a related muscle group would also be expected to be used.

In the examples of Figs. 8A and 8B an association between two features is made. In some examples a network of associated features can be generated. The network of associated features can enable a plurality of components or other features of the biological sample to be connected together.

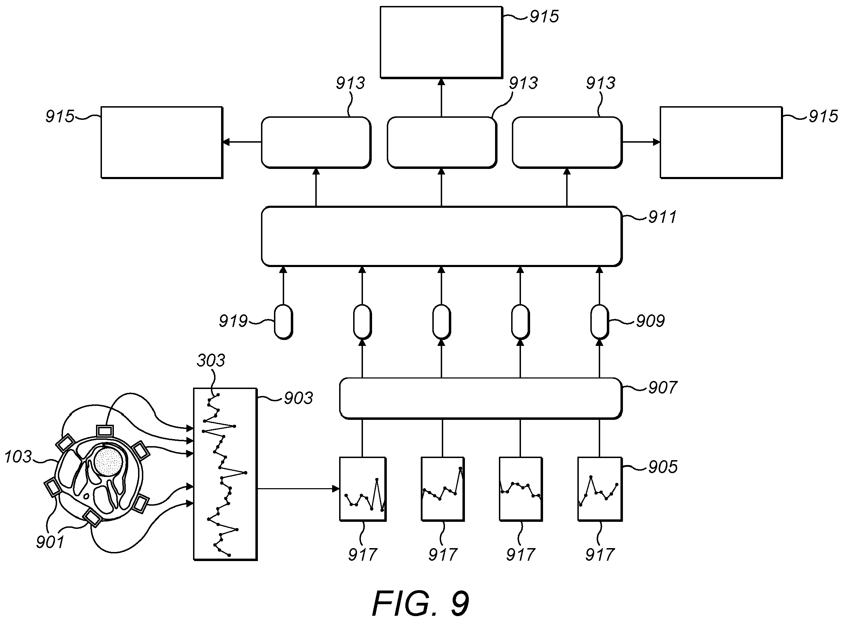

Fig. 9 shows an example method that can be used in some examples of the disclosure.

In this example the biological sample 103 comprises a user's forearm, shown as a cross section view of the major interior anatomical constituents. Other types of biological samples could be used in other examples of the disclosure.

A plurality of electrodes 901 are provided on the biological sample 103 and used to stimulate the biological sample 103. The electrodes 901 are provided on different locations around the biological sample 103. This enables different parts of the biological sample 103 to be analysed. For example, it allows the input signal to be provided to (and the output signal to be collected from) different parts of the biological sample 103.

At block 903 a waveform 303 is obtained from the biological sample 103. The waveforms 303 can comprise a plurality of multi-dimensional and multi-layered datasets. The waveform 303 can comprise information obtained from different parts of the biological sample 103. The waveform 303 can comprise a plurality of features.

At block 905 the method comprises dividing the waveforms 303 into a plurality of sections 917. Each section 917 can comprise at least one feature. The different sections 917 can be processed in parallel. That is each of the different sections 917 can be processed at the same time.

At block 907 the different sections are converted into a format that can be used as an input for an algorithm. For example, the different sections can be converted into a vector input or into any other suitable type of format or representation.

At block 909 different indices can be assigned to the different sections 917 of the waveforms 303. The different indices can enable the different sections to be tracked through the process. The different indices can enable features from different sections 917 to be associated with each other. A reference index 919 can also be provided. The reference index 919 comprises a tag that applies to all of the different sections 917 of the same waveform 303. This enables different sections 917 of the same waveform 303 to be referenced.

At block 911 the different sections 917 can be processed. The processing can comprise, for at least a subset of the sections 917, filtering the waveforms 303 to select features of interest and comparing the selected features of interest to a plurality of reference features. The processing can also comprise making one or more associations between two or more selected features of interest.

At block 913 the associations between the different features of interest can be used to classify the associated features of interest. At block 913 a plurality of associations can be made. The plurality of associations can be made at the same time. The plurality of associations can enable different combinations of the available features of interest to be analysed. These associations can enable a classification to be made. The classification can classify the associations of features as being indicative of a particular feature of interest and/or as not being indicative of a particular feature of interest.

At block 915 one or more outputs can be provided. The outputs that are provided can be based on the classification that is made at block 913. The outputs that are provided can provide an indication of the characteristics of the biological sample 103 that have been identified by the classification at block 913. For example, the output can provide an indication that a particular gesture has been made. This could enable the characteristics of the biological sample 103 to be used to control a machine or to provide inputs to another device or for any other purpose. For example, it can be used to provide an interface between the biological sample 103 and another device. In some examples the output can provide an indication of a condition of the biological sample. This could be used for health monitoring and/or diagnostic purpose. For example, it could provide an indication about the presence or change in particular types of tissue within the biological sample 103.

In the example of Fig. 9, the method comprises a plurality of outputs. This might be the case when a plurality of characteristics are identified within the same biological sample 103. In some examples only a single output might be provided. This might be the case when a single characteristic is identified within the biological sample 103.

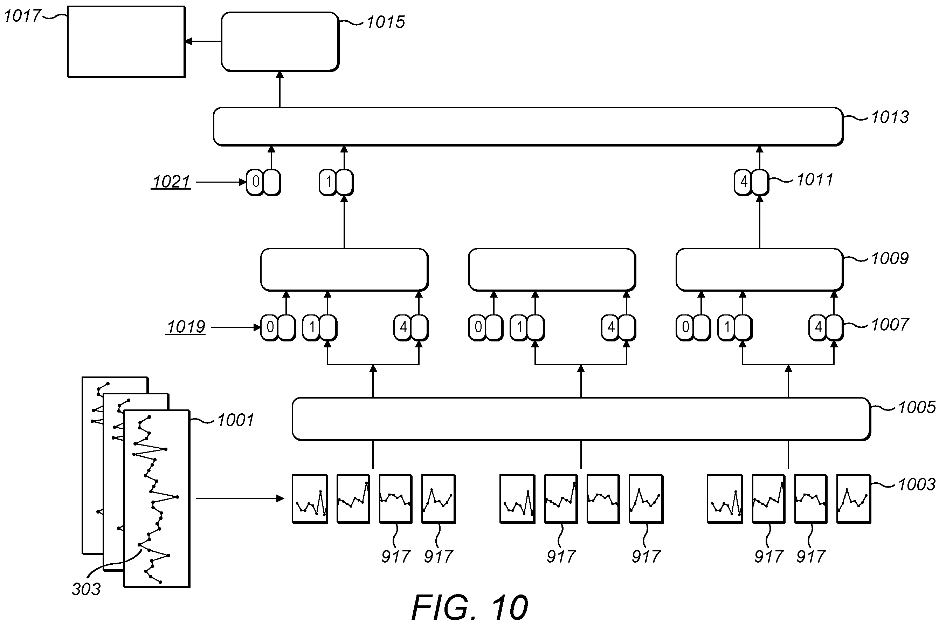

Fig. 10 shows another example method that can be used in some examples of the disclosure. In this example the method can be configured to enable the biological sample 103 to be continuously monitored for detecting features of interest over a period of time. This can enable information obtained at different times to be obtained and processed. In some examples this can enable information obtained at different times to be used to identify one or more characteristics of the biological sample 103. For instance, associations could be made between features that occur within waveforms obtained at different time instances. These associations could be made as well as, or instead of, associations relating to different parts of the biological sample 103.

The biological sample 103 can comprise a user's forearm as shown in Fig. 9 or any other suitable type of biological sample 103.

At block 1001 a plurality of waveforms 303 are obtained from the biological sample 103. The plurality of waveforms 303 can be obtained at different times. In some examples the plurality of waveforms 303 can be obtained by monitoring the biological sample 103 over extended periods of time. The waveforms 303 comprise a plurality of multi-dimensional and multi-layered datasets. In some examples the waveforms 303 can comprise information obtained from different parts of the biological sample 103 as well as information obtained at different times.

Each of the waveforms 303 that are obtained can comprise a plurality of features. Different features can be provided in different waveforms obtained at different times. In some examples the same feature can be provided within two or more of the different waveforms obtained at different times. This can enable changes in the features to be monitored over time.

At block 1003 the method comprises dividing the each of the waveforms 303 into a plurality of sections 917. Each section 917 of each of the waveforms 303 can comprise at least one feature. The different sections 917 of the different waveforms 303 can be processed in parallel. That is each of the different sections 917 of the different waveforms 303 can be processed at the same time.

At block 1005 the different sections are converted into a format that can be used as an input for an algorithm. For example, the different sections 917 can be converted into a vector input or an array input or into any other suitable type of format.

In some examples converting the section into a format that can be used as an input for an algorithm can comprise transforming the data between different domains. For example, data from a time domain can be converted to a frequency domain or to a partial frequency domain.

At block 1007 different indices can be assigned to the different sections 917 of the waveforms 303. This can enable the different sections to be tracked through the process. The different indices can indicate different spatial areas of the biological sample 103. A reference index 1019 can also be provided. The reference index 1019 comprises a tag that applies to all of the different sections 917 of the same waveform 303. This enables different sections 917 of the same waveform 303 to be referenced.

At block 1009 the different sections 917 can be spatially encoded. The processing can comprise, for at least a subset of the sections 917, filtering the waveforms 303 to select features of interest and comparing the selected features of interest to a plurality of reference features. The processing can also comprise making one or more associations between two or more selected features of interest.

At block 1011 different temporal indices can be assigned to the different sections 917 of the waveforms 303. These temporal indices can enable the different temporal sections to be tracked through the process. The different temporal indices can indicate different waveforms obtained at different times from the biological sample 103. Another reference index 1021 can also be provided. The reference index 1021 comprises a tag that applies to all of the different sections 917 of the same temporal waveform 303. This enables different sections 1021 of the same temporal waveform 303 to be referenced.

At block 1013 the different sections 917 are temporally encoded. The temporal encoding can comprise filtering the sections 917 of the waveforms to select different temporal features of interest and comparing the selected features of interest to a plurality of reference features. The processing can also comprise making one or more associations between two or more selected features of interest.

At block 1015 associations between the different features of interest can be used to classify the associated features of interest. The different features of interest can comprise spatial features of interest and temporal features of interest.

At block 1017 one or more outputs can be provided. The outputs that are provided can be based on the classification that is made at block 1015. The output that is provided can provide an indication of the characteristics of the biological sample 103 that have been identified at block 1015. For example, the output can provide an indication that a particular gesture has been made. This could enable the characteristics of the biological sample 103 to be used to control a machine or to provide inputs to another device or for any other purpose. For example, it can be used to provide an interface between the biological sample 103 and another device. In some examples the output can provide an indication of a condition of the biological sample. This could be used for health monitoring and/or diagnostic purpose. For example, it could provide an indication about the presence or change in particular types of tissue within the biological sample 103.

In the example of Fig. 10 the method comprises a plurality of outputs. This might be the case when a plurality of characteristics are identified within the same biological sample 103. In some examples only a single output might be provided.

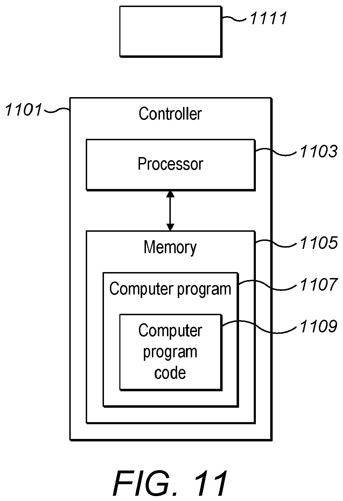

Fig. 11 schematically illustrates an apparatus 101 that can be used to implement examples of the disclosure. In this example the apparatus 101 comprises a controller 1101. The controller 1101 illustrated in Fig. 11 can be a chip or a chip-set. In some examples the controller 1101 can be provided within a computer or other device that can be configured to provide signals and receive signals and/or datasets.

In the example of Fig. 11 the implementation of the controller 1101 can be as controller circuitry. In some examples the controller 1101 can be implemented in hardware alone, have certain aspects in software including firmware alone or can be a combination of hardware and software (including firmware).

As illustrated in Fig. 11 the controller 1101 can be implemented using instructions that enable hardware functionality, for example, by using executable instructions of a computer program 1107 in a general-purpose or special-purpose processor 1103 that can be stored on a computer readable storage medium (disk, memory etc.) to be executed by such a processor 1103.

The processor 1103 is configured to read from and write to the memory 1105. The processor 1103 can also comprise an output interface via which data and/or commands are output by the processor 1103 and an input interface via which data and/or commands are input to the processor 1103.

The memory 1105 is configured to store a computer program 1107 comprising computer program instructions (computer program code 1109) that controls the operation of the controller 1101 when loaded into the processor 1103. The computer program instructions, of the computer program 1107, provide the logic and routines that enables the controller 1101 to perform the methods illustrated in Figs. 2- 3, 6-7 and 9-10. The processor 1103 by reading the memory 1105 is able to load and execute the computer program 1107.

The apparatus 101 therefore comprises: at least one processor 1103; and at least one memory 1105 including computer program code 1109, the at least one memory 1105 and the computer program code 1109 configured to, with the at least one processor 1103, cause the apparatus 101 at least to perform:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

As illustrated in Fig. 11 the computer program 1107 can arrive at the controller 1101 via any suitable delivery mechanism 1111. The delivery mechanism 1111 can be, for example, a machine readable medium, a computer-readable medium, a non-transitory computer-readable storage medium, a computer program product, a memory device, a record medium such as a Compact Disc Read-Only Memory (CD-ROM) or a Digital Versatile Disc (DVD) or a solid state memory, an article of manufacture that comprises or tangibly embodies the computer program 1107. The delivery mechanism can be a signal configured to reliably transfer the computer program 1107. The controller 1101 can propagate or transmit the computer program 1107 as a computer data signal. In some examples the computer program 1107 can be transmitted to the controller 1101 using a wireless protocol such as Bluetooth, Bluetooth Low Energy, Bluetooth Smart, 6LoWPan (IPᵥ6 over low power personal area networks) ZigBee, ANT+, near field communication (NFC), Radio frequency identification, wireless local area network (wireless LAN) or any other suitable protocol.

The computer program 1107 comprises computer program instructions for causing an apparatus 101 to perform at least the following:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

The computer program instructions can be comprised in a computer program 1107, a non-transitory computer readable medium, a computer program product, a machine readable medium. In some but not necessarily all examples, the computer program instructions can be distributed over more than one computer program 1109.

Although the memory 1105 is illustrated as a single component/circuitry it can be implemented as one or more separate components/circuitry some or all of which can be integrated/removable and/or can provide permanent/semi-permanent/ dynamic/cached storage.

Although the processor 1103 is illustrated as a single component/circuitry it can be implemented as one or more separate components/circuitry some or all of which can be integrated/removable. The processor 1103 can be a single core or multi-core processor.

References to "computer-readable storage medium", "computer program product", "tangibly embodied computer program" etc. or a "controller", "computer", "processor" etc. should be understood to encompass not only computers having different architectures such as single /multi- processor architectures and sequential (Von Neumann)/parallel architectures but also specialized circuits such as fieldprogrammable gate arrays (FPGA), application specific circuits (ASIC), signal processing devices and other processing circuitry. References to computer program, instructions, code etc. should be understood to encompass software for a programmable processor or firmware such as, for example, the programmable content of a hardware device whether instructions for a processor, or configuration settings for a fixed-function device, gate array or programmable logic device etc.

As used in this application, the term "circuitry" can refer to one or more or all of the following:
(a) hardware-only circuitry implementations (such as implementations in only analog and/or digital circuitry) and
(b) combinations of hardware circuits and software, such as (as applicable):
   (i) a combination of analog and/or digital hardware circuit(s) with software/firmware and
   (ii) any portions of hardware processor(s) with software (including digital signal processor(s)), software, and memory(ies) that work together to cause an apparatus, such as a mobile phone or server, to perform various functions and
(c) hardware circuit(s) and or processor(s), such as a microprocessor(s) or a portion of a microprocessor(s), that requires software (e.g. firmware) for operation, but the software can not be present when it is not needed for operation.

This definition of circuitry applies to all uses of this term in this application, including in any claims. As a further example, as used in this application, the term circuitry also covers an implementation of merely a hardware circuit or processor and its (or their) accompanying software and/or firmware. The term circuitry also covers, for example and if applicable to the particular claim element, a baseband integrated circuit for a mobile device or a similar integrated circuit in a server, a cellular network device, or other computing or network device.

The apparatus 101 as shown in Fig. 11 can be provided within any suitable device. In some examples the apparatus 101 can be provided within a mobile telephone or any other suitable processing device. The device comprising the apparatus 101 can be coupled to two or more electrodes or other types of sensors that can be configured to detect signals from the biological sample 103 and enable the signals or data representative of the signals to be provide to the device.

The blocks illustrated in Fig. 2 can represent steps in a method and/or sections of code in the computer program 1107. The illustration of a particular order to the blocks does not necessarily imply that there is a required or preferred order for the blocks and the order and arrangement of the blocks can be varied. Furthermore, it can be possible for some blocks to be omitted.

The systems, apparatus 101, methods and computer programs 1107 can use machine learning which can include statistical learning. Machine learning is a field of computer science that gives computers the ability to learn without being explicitly programmed. The computer learns from experience E with respect to some class of tasks T and performance measure P if its performance at tasks in T, as measured by P, improves with experience E. The computer can often learn from prior training data to make predictions on future data. Machine learning includes wholly or partially supervised learning and wholly or partially unsupervised learning. It may enable discrete outputs (for example classification, clustering) and continuous outputs (for example regression). Machine learning may for example be implemented using different approaches such as cost function minimization, artificial neural networks, support vector machines and Bayesian networks for example. Cost function minimization may, for example, be used in linear and polynomial regression and K-means clustering. Artificial neural networks, for example with one or more hidden layers, model complex relationship between input vectors and output vectors. Support vector machines can be used for supervised learning. A Bayesian network is a directed acyclic graph that represents the conditional independence of a number of random variables.

Examples of the disclosure can be used in a range of different applications. For examples in some examples the apparatus can be used to provide an interface between the biological sample 103 and one or more other devices. In such examples a user, or part of a suer, would be the biological sample 103. The user could make gestures or other types of inputs which could be detected and identified by the apparatus employing examples of the disclosure. The apparatus could be configured to distinguish between different types of gestures. This could enable different inputs, or types of input to be made. In such examples the input data could be obtained from sensors provided on any suitable location of the user.

In some examples the apparatus can be configured to detect abnormalities within a biological sample 103. For example, they can be configured to detect ventilation difficulties or an increase in the amount of blood within the biological sample 103. This can be used to screen the biological samples 103 or for any other purpose. For example, information about any abnormalities from the biological sample could be analysed. The examples of the disclosure could enable the type of abnormality to be identified. This information could then be provided to a user or to a medical consultant to enable a diagnosis to be made.

In some examples the apparatus can be used to monitor vital signs of a user. The vital signs could be any parameters of the user that are routinely monitored by medical professionals and/or health care providers. The vital signs that are monitored could comprise temperature, pulse rate, respiration rate, blood pressure and/or any other suitable information. Any changes in the vital signs could then be reported to the user and/or to a health care provider.

In some examples the apparatus could be used to monitor for abnormal growths within the biological sample 103. For example, it could be used to monitor for cancerous growths or other types of growths within the biological sample 103. Examples of the disclosure can enable information about such growths to be obtained without the need for any imaging techniques. That is indications about characteristics of the growths such as size, and location, could be obtained without having to obtain an image of the growth.

The term 'comprise' is used in this document with an inclusive not an exclusive meaning. That is any reference to X comprising Y indicates that X may comprise only one Y or may comprise more than one Y. If it is intended to use 'comprise' with an exclusive meaning then it will be made clear in the context by referring to "comprising only one..." or by using "consisting".

In this description, reference has been made to various examples. The description of features or functions in relation to an example indicates that those features or functions are present in that example. The use of the term 'example' or 'for example' or 'can' or 'may' in the text denotes, whether explicitly stated or not, that such features or functions are present in at least the described example, whether described as an example or not, and that they can be, but are not necessarily, present in some of or all other examples. Thus 'example', 'for example', 'can' or 'may' refers to a particular instance in a class of examples. A property of the instance can be a property of only that instance or a property of the class or a property of a sub-class of the class that includes some but not all of the instances in the class. It is therefore implicitly disclosed that a feature described with reference to one example but not with reference to another example, can where possible be used in that other example as part of a working combination but does not necessarily have to be used in that other example.

Although examples have been described in the preceding paragraphs with reference to various examples, it should be appreciated that modifications to the examples given can be made without departing from the scope of the claims.

Features described in the preceding description may be used in combinations other than the combinations explicitly described above.

Although functions have been described with reference to certain features, those functions may be performable by other features whether described or not.

Although features have been described with reference to certain examples, those features may also be present in other examples whether described or not.

The term 'a' or 'the' is used in this document with an inclusive not an exclusive meaning. That is any reference to X comprising a/the Y indicates that X may comprise only one Y or may comprise more than one Y unless the context clearly indicates the contrary. If it is intended to use 'a' or 'the' with an exclusive meaning then it will be made clear in the context. In some circumstances the use of 'at least one' or 'one or more' may be used to emphasis an inclusive meaning but the absence of these terms should not be taken to infer any exclusive meaning.

The presence of a feature (or combination of features) in a claim is a reference to that feature or (combination of features) itself and also to features that achieve substantially the same technical effect (equivalent features). The equivalent features include, for example, features that are variants and achieve substantially the same result in substantially the same way. The equivalent features include, for example, features that perform substantially the same function, in substantially the same way to achieve substantially the same result.

In this description, reference has been made to various examples using adjectives or adjectival phrases to describe characteristics of the examples. Such a description of a characteristic in relation to an example indicates that the characteristic is present in some examples exactly as described and is present in other examples substantially as described.

Whilst endeavoring in the foregoing specification to draw attention to those features believed to be of importance it should be understood that the Applicant may seek protection via the claims in respect of any patentable feature or combination of features hereinbefore referred to and/or shown in the drawings whether or not emphasis has been placed thereon.

## Claims

1. An apparatus comprising means for:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

2. An apparatus as claimed in claim 1 wherein the plurality of multi-dimensional and multi-layered datasets are obtained from an output signal provided by stimulating the biological sample with one or more of: an electrical signal, an acoustic signal, an electromagnetic signal so as to cause changes in electrical properties of the biological sample.

3. An apparatus as claimed in any preceding claim wherein the plurality of multidimensional and multi-layered datasets comprise electrical impedance tomography signals.

4. An apparatus as claimed in any preceding claim wherein the reference features are determined from calibration outputs from a reference biological sample and the reference features that are determined from calibration outputs are identified as corresponding to one or more characteristics of the biological sample.

5. An apparatus as claimed in claim 4 wherein at least some of the plurality of reference features are associated with each other based on the one or more characteristics of the biological sample that the reference features correspond to.

6. An apparatus as claimed in claim 5 wherein the means are for accessing a memory storing the reference features and information indicative of the one or more associations between the different reference features.

7. An apparatus as claimed in any of claims 5 to 6 wherein different associations between different selected features of interest enable one or more characteristics of the biological sample to be identified.

8. An apparatus as claimed in any preceding claim wherein the reference features are given identifiers that are linked to characteristics of the biological sample and in response to identifying a match between a respective selected feature and a respective reference feature, using the identifiers to identify the one or more characteristics of the biological sample.

9. An apparatus as claimed in any preceding claim comprising means for dynamically tuning one or more filter settings for filtering the plurality of multidimensional and multi-layered datasets to select features of interest.

10. An apparatus as claimed in any preceding claim comprising means for autonomously learning and storing reference features by operating the filters iteratively on labelled reference features.

11. An apparatus as claimed in any preceding claim wherein the means are for processing at least some of the plurality of sections of the datasets in parallel, wherein the processing comprises filtering a plurality of sections of the datasets to select features of interest and wherein the parallel processing of the plurality of sections enables a plurality of different features of interest to be selected simultaneously.

12. An apparatus as claimed in any preceding claim wherein the plurality of multidimensional and multi-layered datasets vary over time and the means are for filtering the plurality of multi-dimensional and multi-layered datasets over a period of time.

13. An apparatus as claimed in any preceding claim wherein the characteristic of the biological sample that is identified comprises one or more of: the position of one or more components within the biological sample, movement of one or more components within the biological sample, the size of one or more components within the biological sample, the conductivity of one or more components within the biological sample, an electrical parameter of one or more components within the biological sample or the shape of one or more components within the biological sample.

14. A method comprising:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.

15. A computer program comprising computer program instructions that, when executed by processing circuitry, cause:
obtaining a plurality of multi-dimensional and multi-layered datasets from a biological sample;
dividing the plurality of multi-dimensional and multi-layered datasets into a plurality of sections wherein each section comprises at least one feature;
for at least a subset of the sections, filtering the plurality of multi-dimensional and multi-layered datasets to select features of interest, comparing the selected features of interest to a plurality of reference features and making one or more associations between two or more selected features of interest to identify one or more characteristics of the biological sample.
